Europäisches Patentamt

European Patent Office

Office européen des brevets

(11) Veröffentlichungsnummer: **0 305 726 B1**

(19)

# EUROPÄISCHE PATENTSCHRIFT

(12)

(45) Veröffentlichungstag der Patentschrift: **25.03.92**

(21) Anmeldenummer: **88112000.0**

(22) Anmeldetag: **26.07.88**

(51) Int. Cl.⁵: **A61K 31/505**, A61K 47/00, A61K 47/12, A61L 15/44

(54) **Transdermales therapeutisches System.**

(30) Priorität: **02.09.87 DE 3729299**

(43) Veröffentlichungstag der Anmeldung:
**08.03.89 Patentblatt 89/10**

(45) Bekanntmachung des Hinweises auf die
Patenterteilung:
**25.03.92 Patentblatt 92/13**

(84) Benannte Vertragsstaaten:
**AT BE CH DE ES FR GB IT LI NL SE**

(56) Entgegenhaltungen:
**EP-A- 0 127 468**
**EP-A- 0 169 364**

(73) Patentinhaber: **Horstmann, Michael Dr.**
**Deichstrasse 9**
**W-5450 Neuwied 1(DE)**

(72) Erfinder: **Guse, Günter, Dr.**
**Liliencronstrasse 30**
**W-2000 Hamburg 73(DE)**
Erfinder: **Horstmann, Michael, Dr.**
**Deichstrasse 9**
**W-5450 Neuwied 1(DE)**
Erfinder: **Erler, Axel**
**Eppendorfer Weg 125**
**W-2000 Hamburg 20(DE)**

## Beschreibung

Die Erfindung betrifft ein transdermales therapeutisches System (TTS) zur Applikation von Moxonidin.

Transdermale Arzneimittel sind bekannt. Auch die prinzipiellen Vorteile, die diese Darreichungsform speziell für systemisch wirkende Arzneistoffe haben kann, sind unumstritten, wie beispielsweise Entlastung des Magen-Darm-Trakts, Erhöhung der Bioverfügbarkeit oder Vermeidung unerwünschter Blutspiegel-Spitzenwerte.

Andererseits ist die Durchlässigkeit der Haut für Arzneistoffe begrenzt. Es ist eher die Ausnahme als die Regel, daß ein Arzneistoff aus einer akzeptabel kleinen Applikationsfläche in solcher Menge durch die Hornschicht der Haut diffundiert, daß therapeutisch relevante Blutspiegel erzeugt werden. Eine solche Ausnahme ist das Glyceroltrinitrat, dessen transdermale Verabreichung aus einem Pflaster bekannt und unter anderem in der DE A 3.518.707 beschrieben ist.

Es hat nicht an Versuchen gefehlt, auch andere Arzneistoffe transdermal zu verabreichen, beispielsweise in Form von Salben, Cremes, Sprays oder als Pflaster. Von einigen wenigen Ausnahmen abgesehen, werden dabei große Applikationsflächen benötigt, die einer bequemen und sicheren Anwendung entgegenstehen und damit letztlich die Therapietreue beeinträchtigen können.

Auch der zentral wirksame Blutdrucksenker Moxonidin, chemisch 4-Chlor-5-(imidazolin-2-ylamino) -6-methoxy-2-methylpyrimidin, dessen transdermale Arzneiform in der EP-A 169 364 beschrieben ist, erfordert nach diesem Stand der Technik eine außerordentlich große Pflasterfläche.

Es ist nun verschiedentlich vorgeschlagen worden, die Durchlässigkeit der Haut durch Einsatz von pharmazeutischen Hilfsstoffen in gewünschter Weise zu erhöhen. Sie werden häufig Permeationsbeschleuniger, Penetrationsförderer, penetration enhancers, im folgenden kurz Enhancer genannt.

Nach dem Stand der Technik werden als Enhancer genannt:

Alkohole in DE-B 2.135.533, DE-A-3.205.258, EP-A 182.635 und EP-A 131.228, aliphatische Ester in US-A-4,336,243, DE-B-2.135.533 und US-A-4,390,520 und Amide in US-A-3,742,951, DE-B-2.604.718 und EP-A 95.169, Glycerinester in US-A-4,336,243 und US-A-4,552,751 und Sulfoxide in DE-B-2.755.661 und DE-A 3.614.843.

Auch organische Säuren wurden als Penetrationsverbesserer insbesondere zusammen mit Alkoholen bzw. Glykolen beschrieben von E.R. Cooper et al.; J. Pharm. Sci. 74, 688 (1985), sowie in DE-A 3.528.979 und EP-A 171.742.

Experimentelle Belege dafür, daß die vorgenannten Enhancer in gewünschter Weise wirken, d.h. bei akzeptabel kleinen Anwendungsflächen zu therapeutisch relevanten, gegenüber dem enhancerfreien System signifikant erhöhten Permeationen führen, fehlen in der Regel. Bei einer Anwendung als Pflaster vom Matrix-Typ, wie sie häufig beansprucht wird, besteht außerdem die Schwierigkeit, pharmazeutische und klebtechnische Erfordernisse gleichermaßen berücksichtigen zu müssen, sowie qualitätsbeständige und sichere Produktion, Lagerung und Anwendung der fertigen Arzneiform, schließlich auch die physiologische Unbedenklichkeit der verwendeten Enhancer im Zusammenspiel mit Klebmasse etc. zu gewährleisten.

Aufgabe der vorliegenden Erfindung war es somit, für Moxonidin als Arzneistoff mit begrenzter Hautgängigkeit ein transdermales therapeutisches System zu entwickeln, das die Nachteile von Produkten nach dem Stand der Technik nicht aufweist und bei dem zudem

a) die Verzögerungszeit bis zum Eintritt des Wirkstoffs in den Kreislauf verkürzt ist,

b) die von da an zu messende Resorptionsrate pro Einheitsfläche erhöht ist und

c) die Resorptionsrate des Wirkstoffs nach Ablauf der Verzögerungszeit (lag time) weitgehend konstant bleibt.

Außerdem sollte das gesamte System physiologisch unbedenklich sein.

Anders ausgedrückt, bestand die Aufgabenstellung darin, einen relativ polaren Arzneistoff, nämlich Moxonidin mit erhöhter Flußrate je Zeit- und Flächeneinheit durch das stratum corneum zu bringen. Dessen Permeationseigenschaften werden wesentlich von den hydrophoben geordneten Strukturen der micellären "Kittphase" dieser Schicht bestimmt. Es war für den Fachmann naheliegend, solche Stoffe als Enhancer in Betracht zu ziehen, die eine gewisse Lipidähnlichkeit aufweisen, also beispielsweise Triglyceride oder Phosphatide. Dies hatte hingegen in der Praxis nicht den gewünschten Erfolg.

Überraschenderweise wurde aber gefunden, daß ein gänzlich anderer Substanztyp, nämlich höhere n-Alkancarbonsäure mit mindestens 12 C-Atomen besonders geeignete Enhancer für den aminischen Arzneistoff Moxonidin sind.

Bevorzugte n-Alkancarbonsäuren sind solche mit 12-18 C-Atomen, vor allem Myristinsäure, Palmitinsäure und ganz besonders Laurinsäure.

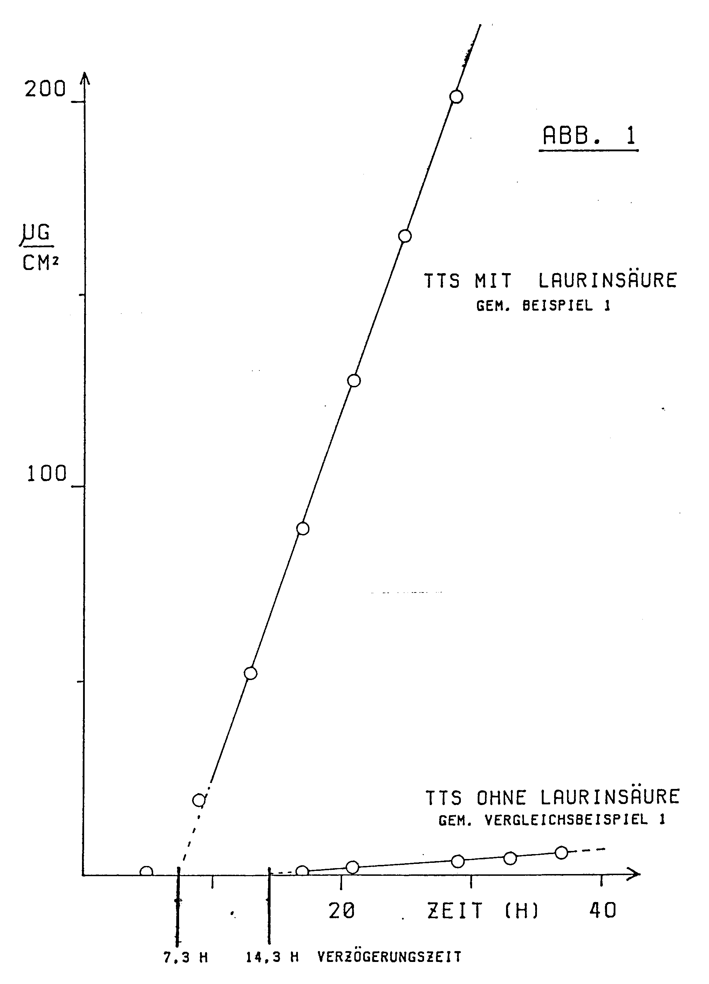

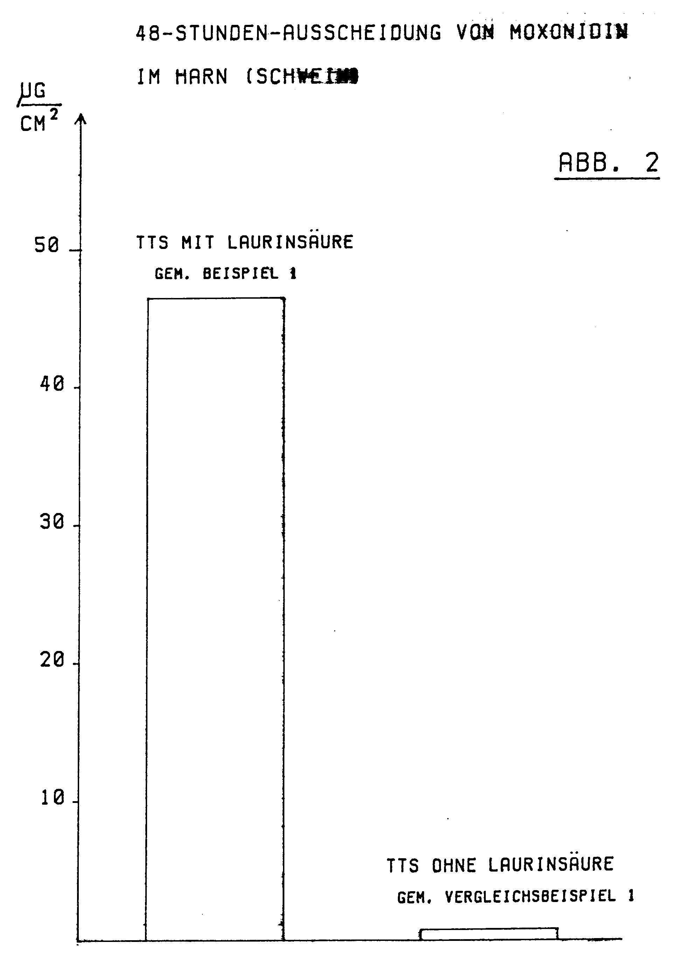

Laurinsäure verkürzt die Verzögerungszeit (lag time) beim Moxonidin gegenüber dem entsprechenden enhancer-freien System in vitro auf etwa die Hälfte (vgl. Abbildung 1). Danach tritt ein stationärer Zustand (steady state) ein; die Freisetzungskurve wird zur Graden. Der transdermale Fluß ist etwa um den Faktor 35 erhöht. Beim Versuch in vivo (Abbildung 2) bestätigt sich dieses Bild.

Das erfindungsgemäße TTS ist überaus geeignet, einen gleichmäßigen transdermalen Fluß über mehrere Tage, insbesondere 3 und mehr Tage, aus einem einzigen Pflaster zu erzeugen. Damit wird eine mehrtägige Behandlung von Bluthochdruck möglich, ohne das Pflaster wechseln zu müssen.

Die verwendeten n-Alkancarbonsäuren, insbesondere Laurinsäure (n-Dodecansäure), Myristinsäure (n-Tetradecansäure) und Palmitinsäure (n-Hexadecansäure), vor allem aber Laurinsäure, nehmen in der Tat als erfindungsgemäße Enhancer eine Sonderstellung ein. Denn nicht nur die genannten Enhancer anderer chemischer Struktur sind für die erfindungsgemäßen Zwecke ungeeignet. Auch die kürzeren Fettsäuren des verwendeten Typs sind für das erfindungsgemäße TTS wenig geeignet, weil mit ihnen die Förderung der Hautpenetration mit der Zeit immer weiter zunimmt und nicht ins Gleichgewicht kommt, so daß mit ihnen eine gleichmäßige Penetrationsrate nicht zu erreichen ist.

Erfindungsgemäße transdermal wirksame Pflaster lassen sich sowohl als Reservoirsysteme mit Kontrollmembran wie auch als Matrixsysteme gestalten, bei denen eine Selbstklebemasse Wirkstoff und Enhancer bereits enthält. Wegen ihres einfachen Aufbaues und der dadurch bedingten hohen Produktions-und Arzneimittelsicherheit werden erfindungsgemäß die letzteren bevorzugt.

In dem erfindungsgemäßen TTS werden im Fall von Pflastern, die vor Salben, Cremes oder Sprays bevorzugt sind, bevorzugt je Quadratmeter etwa 5 - 10 g Moxonidin verwendet, sowie etwa 7 - 20 g n-Alkancarbonsäure, wie Laurinsäure, Myristinsäure oder Palmitinsäure. Falls erwünscht, können dabei die erfindungsgemäß verwendeten Säuren einzeln oder im Gemisch verwendet werden. Dabei ist bevorzugt Laurinsäure überwiegend vorhanden, insbesondere mit mehr als 50 Gew.-% der verwendeten Enhancer. Besonders bevorzugt ist ein Gehalt von etwa 5 - 10 g Moxonidin und 7 - 15 g Laurinsäure je Quadratmeter. Weiterhin bevorzugt beträgt das Gewichtsverhältnis von Moxonidin zu Enhancer in einem erfindungsgemäßen TTS, insbesondere Pflaster, 1:1 bis 1:2.

Die Auswahl der Basis-Selbstklebemassen für erfindungsgemäße transdermal wirksame Pflaster ist nicht kritisch. Jede Selbstklebemasse ist geeignet, die die geforderten klebtechnischen Eigenschaften aufweist, mit Wirkstoff und Enhancer nicht in unerwünschte Wechselwirkung tritt und beide Stoffe in der oben beschriebenen Weise freisetzt. Mit besonderem Vorteil werden Polyacrylat-Selbstklebemassen und solche aus gesättigten Kautschuken und Klebharzen verwendet, beispielsweise auf Basis von Polyisobuten und synthetischen Kohlenwasserstoffharzen.

Das Einarbeiten von Wirkstoff und Enhancer in das Basispolymer kann durch einfaches Mischen mit einem Rührwerk erfolgen, wird aber mit besonderem Vorteil in einer Kugelmühle nach dem Verfahren der DE-A-36 17 158 durchgeführt. Diese Behandlung führt zu einer erheblichen Verbesserung der Kornfeinheit und Benetzung von Wirkstoff und Hilfsstoff und damit zu erhöhter Homogenität des Gesamtsystems.

Die verwendeten Fettsäuren sind sehr wirksame Weichmacher für Selbstklebemassen, was bei Verwendung von an sich schon weichen Basispolymeren zum Fadenziehen und zu unerwünschten Kleberrückständen auf der Haut führen kann. Bei Polyacrylaten läßt sich die Cohäsion der Matrix bei Bedarf ohne Schwierigkeiten durch Vernetzung erhöhen, besonders vorteilhaft durch UV-Bestrahlung gemäß DE-B-2.743.979, bei Polyisobutenen durch Verwendung besonders hochmolekularer Typen.

Als Trägermaterialien für die selbstklebenden wirkstoff-und enhancerhaltigen Pflaster können sowohl occlusiv wirkende Folien oder dergleichen als auch luft- und wasserdampfdurchlässige Gewebe, Vliese oder dergleichen verwendet werden.

Das Beschichten des Trägermaterials, das Trocknen, das Abdecken mit abhäsiver Schutzfolie sowie das Ausstanzen und Verpacken einzelner Pflaster kann nach den üblichen Methoden der Fertigung medizinischer Pflaster erfolgen.

Die Erfindung wird nachstehend durch Beispiele weiter erläutert.

Beispiel 1

Ein Basispolymeres aus
18,5 Gew.-% n-Butylacrylat
78,2 Gew.-% 2-Ethylhexylacrylat
2,8 Gew.-% Acrylsäure
0,5 Gew.-% Benzoinacrylat
wird durch radikalische Lösungspolymerisation nach DE-PS 2.743.979 hergestellt.

65 g dieses Basispolymeren, gelöst in Benzin/Aceton, 15 g mikronisierte Moxonidin-Base und 20 g Laurinsäure werden in eine Hartporzellan-Kugelmühle gegeben und der Gesamtfestkörpergehalt mit Ethylacetat auf 35 Gew.-% eingestellt. Die verschlossene Kugelmühle rotiert 24 h mit ca. 25 U/min auf der Rollbank. Anschließend kann die homogenisierte Zubereitung als streichfertige Selbstklebemasse entnommen werden. Sie wird in einer solchen Schichtdicke auf Polyesterfolie von 15 Micrometer Stärke gestrichen, daß nach 48 h Trocknung bei Raumtemperatur eine Schicht von 50 g/m$^2$ verbleibt. Sie wird mit abhäsiver Schutzfolie eingedeckt.

Die so hergestellte selbstklebende Matrix enthält 7,5 g Moxonidin und 10 g Laurinsäure je Quadratmeter und kann in Pflaster beliebiger Größe geschnitten werden.

Zur Prüfung der erfindungsgemäßen Pflaster werden die nachstehend beschriebenen Methoden angewendet.

Penetration durch exzidierte Schweinehaut.

Ein kreisförmiges Stück Schweinehaut - ca. 7 cm Durchmesser - wird von Resten des subkutanen Fett- und Bindegewebes befreit. Das Hautstück wird nun außenseitig mit einem von der Schutzfolie befreiten Pflaster (16 cm$^2$ Fläche) beklebt. In einer geeigneten Glasapparatur wird in einem Wasserbad mit 34,0 +-0,1 °C der Durchtritt der Wirksubstanz durch die Tierhaut in eine Phosphatpufferlösung (pH 5,5) untersucht. Dabei werden im Abstand von wenigen Stunden Proben der Pufferlösung entnommen, in denen mittels Hochdruckflüssigkeitschromatographie der Wirkstoffgehalt und damit die pro Zeit und Fläche aufgenommene Substanzmenge bestimmt wird. Das Volumen der Pufferlösung wird so eingestellt, daß über die ganze Versuchsdauer (ca. 40 Stunden) "sink"-Bedingungen herrschen.

Abbildung 1 zeigt die von der Pufferlösung flächenspezifisch aufgenommene Menge Moxonidin gegenüber dem Vergleichsbeispiel 1.

Bestimmung der Bioverfügbarkeit am Tier.

Einem schwach behaarten Schwein wird in der Lendengegend ein 16 cm$^2$-Pflaster aufgeklebt und für die gesamte Versuchsdauer an Ort und Stelle belassen.

Die im Harn des Tieres mittels Hochdruckflüssigkeitschromotographie gemessene Moxonidin-Konzentration wird auf das im Sammelintervall von 24 Stunden abgegebene Volumen bezogen. Da Moxonidin fast ausschließlich unverändert über die Niere ausgeschieden wird, errechnet sich hierbei die in Abbildung 2 gegenüber dem Vergleichsbeispiel 1 dargestellte Resorptionsrate pro Quadratzentimer Pflaster.

Beispiel 2

Eine Basismischung aus

| | |
|---|---|
| 31,8 Gew.-% | Polyisobuten $M_v$ = 2.800.000 (Oppanol B® 150) |
| 40,9 Gew.-% | Polyisobuten $M_v$ = 40.000 (Oppanol B® 10) |
| 18,2 Gew.-% | aliph. Kohlenwasserstoffharz mit Erweichungspunkt R + K 97 °C (Escorez® 1202) |
| 9,1 Gew.-% | dickflüssigem Paraffinöl (Goldöl®) |

in n-Heptan wird im Kneter bereitet.

65 g Festkörper dieser Basismischung, 15 g mikronisierte Moxonidin-Base und 20 g Laurinsäure werden in eine Hartporzellan-Kugelmühle gegeben und der Gesamtfestkörpergehalt mit n-Heptan/Ethylacetat 9:1 auf 33 Gew.-% eingestellt.

Weitere Verarbeitung und Prüfung erfolgen gemäß Beispiel 1.

Vergleichsbeispiel 1

Eine Mischung von 81,25 g des Basispolymeren gemäß Beispiel 1, gelöst in Benzin/Aceton, und 18,75 g mikronisierter Moxonidin-Base wird wie in Beispiel 1 hergestellt, vermahlen, mit 40 g/m$^2$ ausgestrichen, getrocknet und geprüft.

Die Mengen sind so abgestimmt, daß wie in Beispiel 1 7,5 g Moxonidin je Quadratmeter vorliegen und auch das Verhältnis von Moxonidin zu Basispolymer das gleiche ist wie dort (1:4,33).

Die Bestimmung der Freisetzung in vitro und in vivo erfolgt wie bei Beispiel 1. Die Ergebnisse zeigen die Abbildungen 1 und 2.

**Patentansprüche**

1. Transdermales therapeutisches System zur Applikation von Moxonidin, enthaltend mindestens eine höhere n-Alkancarbonsäure mit mindestens 12 C-Atomen als Permeationsbeschleuniger.

2. Transdermales therapeutisches System nach Anspruch 1, dadurch gekennzeichnet, daß das System ein selbstklebendes Pflaster vom Matrix-Typ ist.

3. Transdermales therapeutisches System nach Anspruch 2, dadurch gekennzeichnet, daß es eine Selbstklebemasse auf Basis eines ggf. vernetzten Acrylats oder auf Basis von Polyisobutenen und synthetischen Kohlenwasserstoffharzen enthält.

4. Transdermales therapeutisches System nach Anspruch 2, dadurch gekennzeichnet, daß es Moxonidin und Laurinsäure enthält.

5. Transdermales therapeutisches System nach Anspruch 4, dadurch gekennzeichnet, daß das Moxonidin mikronisiert ist.

6. Transdermales therapeutisches System nach Anspruch 4, dadurch gekennzeichnet, daß es auf je ein Gewichtsteil Moxonidin 1 - 2 Gewichtsteile Laurinsäure enthält.

7. Transdermales therapeutisches System nach Anspruch 2, dadurch gekennzeichnet, daß es je Quadratmeter 5 - 10 g Moxonidin und 7 -20 g Laurinsäure, insbesondere 5 - 10 g Moxonidin und 7 - 15 g Laurinsäure enthält.

**8.** Verfahren zur Herstellung eines transdermalen therapeutischen Systems nach einem der Ansprüche 1 - 7, dadurch gekennzeichnet, daß man Moxonidin mit mindestens einer n-Alkancarbonsäure mit mindestens 12 C-Atomen in fein verteilter Form in das System einarbeitet.

**Claims**

**1.** A transdermal therapeutic system for the administration of moxonidine, comprising moxonidine and at least one higher n-alkane carboxylic acid with at least 12 C-atoms as a permeation enhancer.

**2.** A transdermal therapeutic system according to claim 1, in the form of a self-adhesive matrix-type patch.

**3.** A transdermal therapeutic system according to claim 2, comprising a self-adhesive composition based on an optionally cross-linked acrylate or based on polyisobutenes and synthetic hydrocarbon resins.

**4.** A transdermal therapeutic system according to claim 2, comprising moxonidine and lauric acid.

**5.** A transdermal therapeutic system according to claim 4, wherein the moxonidine is micronized.

**6.** A transdermal therapeutic system according to claim 4, comprising 1 to 2 parts by weight of lauric acid per part by weight of moxonidine.

**7.** A transdermal therapeutic system according to claim 2, comprising 5 to 10 g of moxonidine and 7-20 g of lauric acid per square meter, in particular 5 to 10 g of moxonidine and 7-15 g of lauric acid.

**8.** Procedure of manufacture of a transdermal therapeutic system according to one of the claims 1 to 7, characterized in that moxonidine is embodied with at least one n-alkane carboxylic acid with at least 12 C-atoms in a finely divided form.

**Revendications**

**1.** Système thérapeutique transdermique pour l'application de moxonidine comprenant au moins un acide d'alcane carbonique à structure moléculaire (n) élevée avec au moins 12 atomes de carbone comme activateurs de pénétration.

**2.** Système thérapeutique transdermique selon la revendication 1., en ceci caractéristique que ce système constitue un emplâtre auto-adhésif de type matrice.

**3.** Système thérapeutique transdermique selon la revendication 2., en ceci caractéristique qu'il contient une masse autoadhésive à base d'acrylate, selon le cas réticulé ou non, ou à base de polyisobutènes et de résines d'hydrocarbures synthétiques.

**4.** Système thérapeutique transdermique selon la revendication 2., en ceci caractéristique qu'il comprend de la moxonidine et un acide laurique.

**5.** Système thérapeutique transdermique selon la revendication 4., en ceci caractéristique que la moxonidine est micronisée.

**6.** Système thérapeutique transdermique selon la revendication 4., en ceci caractéristique qu'il comprend une à deux parts pondérales d'acide laurique pour chaque part pondérale de moxonidine.

**7.** Système thérapeutique transdermique selon la revendication 2., en ceci caractéristique que chaque mètre carré comprend de 5 à 10 grammes de moxonidine et 7 à 20 grammes d'acide laurique, en particulier 5 à 10 grammes de moxonidine et 7 à 15 grammes d'acide laurique.

**8.** Méthode de production d'un système thérapeutique transdermique selon les revendications 1 à 7, en ceci caractéristique que l'on intègre au système de la moxonidine avec au moins un acide d'alcane carbonique à structure moléculaire (n) élevée avec au moins 12 atomes de carbone sous forme d'une répartition fine.

ABB. 1

TTS MIT LAURINSÄURE
GEM. BEISPIEL 1

TTS OHNE LAURINSÄURE
GEM. VERGLEICHSBEISPIEL 1

$\frac{UG}{CM^2}$

200

100

20    ZEIT (H)    40

7,3 H    14,3 H    VERZÖGERUNGSZEIT

48-STUNDEN-AUSSCHEIDUNG VON MOXONIDIN

IM HARN (SCHWEIN)

ABB. 2

$\dfrac{\mu G}{CM^2}$

TTS MIT LAURINSÄURE

GEM. BEISPIEL 1

50

40

30

20

10

TTS OHNE LAURINSÄURE

GEM. VERGLEICHSBEISPIEL 1